# EUROPEAN PATENT APPLICATION

(11) **EP 3 912 996 A1**
(43) Date of publication of application: **24.11.2021**
(21) Application number: 20315244.2
(22) Date of filing: 18.05.2020
(51) Int. Cl.: C07K 16/24, A61P 31/00, A61K 39/00

(54) **TREG EXPRESSING TNF RECEPTOR TYPE 2 PLAY A MAJOR ROLE IN CD4+ T CELL IMPAIRMENT DURING ACUTE POST-STRESS IMMUNODEPRESSION**

(71) Applicant: Université de Nantes, 44000 Nantes (FR); CHU de Nantes - Centre Hospitalier Universitaire de Nantes, 44000 Nantes (FR); INSERM (Institut National de la Santé et de la Recherche Médicale), 75013 Paris (FR); Centre National de la Recherche Scientifique, 75016 Paris (FR); Sorbonne Université, 75006 Paris (FR)
(72) Inventor: Asehnoune, Karim, 44000 Nantes (FR); Gaborit, Benjamin, 44400 Nantes (FR); Roquilly, Antoine, 44300 Nantes (FR); Louvet, Cédric, 41100 Nantes (FR); Salomon, Benoît, 94100 Saint-Maur (FR)
(74) Representative: Novagraaf Technologies

(57) **Abstract**

The present invention concerns an inhibiting T regulatory (Treg) cell therapy consisting of a tumor necrosis factor receptor 2 (TNFR2) antagonist and/or a composition enriched in lymphocytes and depleted of Treg cells, for use in the prevention or treatment of an acute post-stress immunodepression, in particular sepsis.

## Description

### Technical field of the invention

The present invention concerns an inhibiting T regulatory (Treg) cell therapy consisting of a tumor necrosis factor receptor 2 (TNFR2) antagonist and/or a composition enriched in lymphocytes and depleted of Treg cells, for use in the prevention or treatment of an acute post-stress immunodepression, in particular sepsis.

In the description below, references in square brackets ([ ]) refer to the list of references at the end of the text.

### State of the art

Sepsis occurs when an infection leads to a systemic inflammatory response with organ failure, which can lead to septic shock (organ failure and hypotension) and death [1]. Sepsis remains the leading cause of death in intensive care units and in hospitals causing more than 5 millions deaths worldside. A significant improvement has been observed in early survival rates with an increasing concern for secondary acquired infections associated with late mortality. Indeed the majority of patients who survive the early systemic inflammatory response of sepsis develop a sustained compensatory anti-inflammatory response that is actually a protracted immunosuppressive state that may last for years [2] [3]. During the immunosuppressive phase of sepsis, current data indicate that bacterial pneumonia (first cause of secondary infections) occurs in 10%-30% of sepsis patients. Interestingly, it has been assumed that the immune function did not recover in those patients who died during the late phase of sepsis [4]. Surprisingly, and despite hundreds of therapeutic clinical studies performed in humans, no FDA-approved specific therapeutic options are available. Therefore the question to be addressed is why sepsis-induced immunosuppression renders the septic patient prone to secondary pneumonia.

The elevation of TNF-α is a hallmark of severe sepsis explaining why anti-TNF therapies targeting TNF or TNFR1 were tested in septic shock in an attempt to blunt the initial overwhelming inflammatory response [5,6]. These studies failed to show any clinical benefit probably because, blocking TNFR1 on effector CD4+ T cells was difficult considering the high rate of apoptosis and T cell exhaustion. Moreover, the anti-inflammatory effects of these therapies may have aggravated immunosuppression.

Severe alteration of the lymphocyte compartment is also a hallmark signature of sepsis induced immune suppression [7,8]. One of the prevailing theories explaining that lymphopenia induces poor outcomes in septic patients relies on the occurrence of secondary nosocomialinfections caused by this sepsis-induced immune impairment [9]. FoxP3+ regulatory T cells (Treg) are good candidates to explain the impairment of the CD4+ T cell compartment observed during sepsis owing to their high suppressive function [10,11]. Treg were found to induce tolerogenic dendritic cells and increase susceptibility to secondary pneumonia following a severe primary infection [12]. However, the mechanisms of early Treg activation during sepsis and whether and how these cells may be responsible for CD4+ T cell depletion, and in that case, what subset of Treg may be involved, is still unknown to date. In cancer, Oppenheim and his team were the first authors to demonstrate the particular immunosuppressive capacities of the TNFR2pos Tregcells population in mice [13]. Blocking TNFR2 with monoclonal antibodies impairs Treg cell accumulation in tumors [14] suggesting a potential for treatment.

But even if the TNFR2 subset of Treg cells is considered to be highly suppressive in chronic human pathologies like cancer [13], no evidence is available to date in acute diseases like sepsis. However, the immune response to sepsis or to chronic human pathologies (like cancer) is very distinct. Indeed, sepsis is characterized by an excessive systemic inflammation (a major cytokine storm) developing over few hours which TNF is the master mediator [15]. The complex mechanisms of the immune response make it a very specific disease [16]. The immunological consequences were also very distinct, as during cancer Treg activation is associated with metastatic dissemination, whereas after sepsis, an increased risk of developing infectious diseases such as pneumonia is observed.

### Description of the invention

The inventors have observed *in vivo* in a mouse model of *Staphylococcus aureus* septicemia and in human Treg during sepsis, that the early maintenance and activation of Treg cells (*i.e*. high suppressive profile) during *Staphylococcus aureus* sepsis induces CD4+T cells impairment and increases susceptibility to secondary pneumonia.

The inventors have identified that among Treg, the TNFR2pos Treg subset endorsed the majority of effector, immunosuppressive functions, and that TNFR2 was particularly associated with activation of genes involved in cell cycle and replication in Treg cells, probably explaining their maintenance. Blocking or deleting TNFR2 during sepsis decreased the susceptibility to secondary infection. In humans, the inventors' data paralleled those in mice, and it was found that the expression of CTLA-4 was dramatically increased in TNFR2pos Treg cells after cultured *in vitro* with *Staphylococcus aureus* and during sepsis (data IBIS-sepsis biocollection). The inventors' findings describe *in vivo* mechanisms underlying sepsis-induced immunosuppression and identify the TNFR2pos Treg subset as a target for therapeutic intervention.

Therefore an object of the present invention concern a tumor necrosis factor receptor 2 (TNFR2) antagonist and/or composition enriched in lymphocytes and depleted of T regulatory (Treg) cells, for use in the prevention or treatment of an acute post-stress immunodepression.

According to a particular embodiment of the present invention, the acute post-stress immunodepression is sepsis.

According to a particular embodiment of the present invention, the TNFR2 antagonist for use according to the present invention, is selected from the group consisting of a blocking anti-TNFR2 antibody or an antigen-binding fragment thereof having an antagonistic effect on TNFR2 upon binding.

According to a particular embodiment of the present invention, the anti-TNFR2 antibody for use according to the present invention, is a monoclonal antibody, a chimeric antibody, a humanized antibody.

According to a particular embodiment of the present invention, the antigen-binding fragment for use according to the present invention, is one or more fragments of an anti-TNFR2 antibody that retain the ability to specifically bind to TNFR2. For example, it is a Fab, Fab', F(ab)'2, scFv, (scFv')2, diabody, triabody, VH or VL domain.

According to a particular embodiment of the present invention, the composition enriched in lymphocytes and depleted of Treg cells for use according to the present invention can be obtained by any method known in the art. For example, it is obtained by contacting *in vitro* a population of human cells comprising said lymphocytes from a human blood or bone marrow sample from a patient having sepsis with a TNFR2 antagonist that suppresses proliferation of Treg cells, thereby producing said composition enriched in lymphocytes and depleted of Treg cells. Preferably, the lymphocytes are obtained from a patient's own blood or bone marrow (autologous cells). For example, the composition for use according to the present invention comprises less than 10%, preferably less than 5%, of Treg cells.

According to a particular embodiment of the present invention, the composition enriched in lymphocytes and depleted of Treg cells for use according to the present invention, is depleted of TNFR2pos Treg cells.

### Brief description of the figures

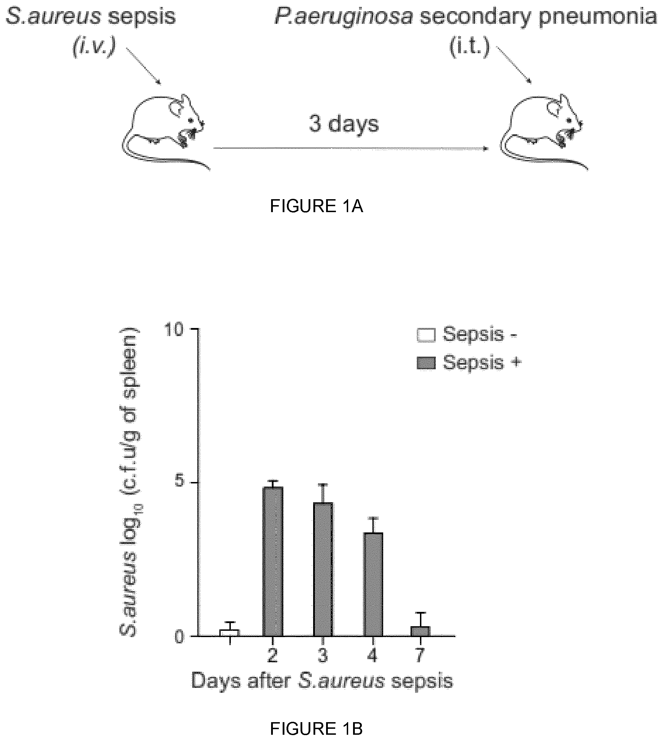

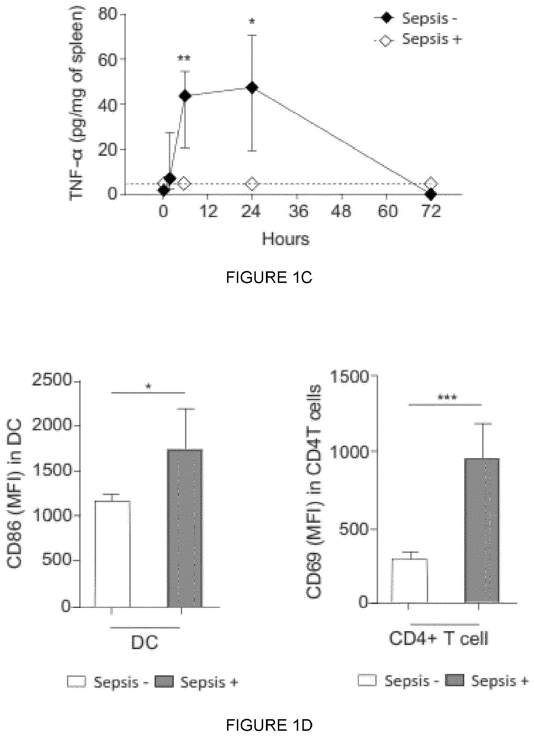

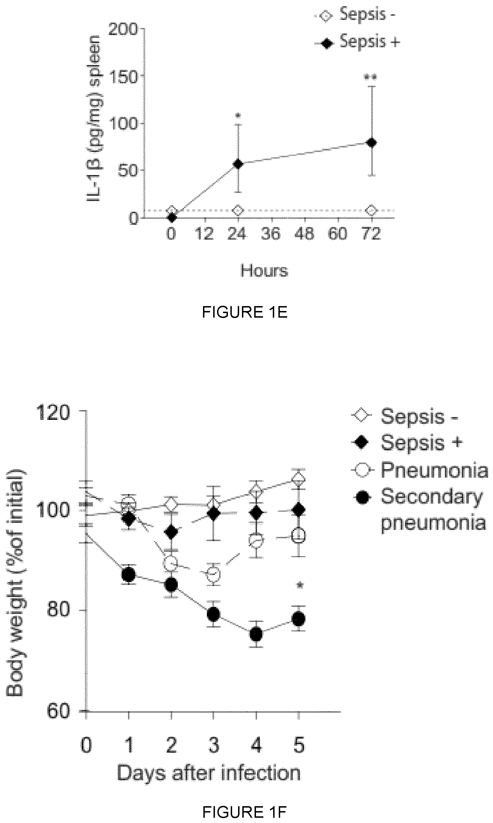

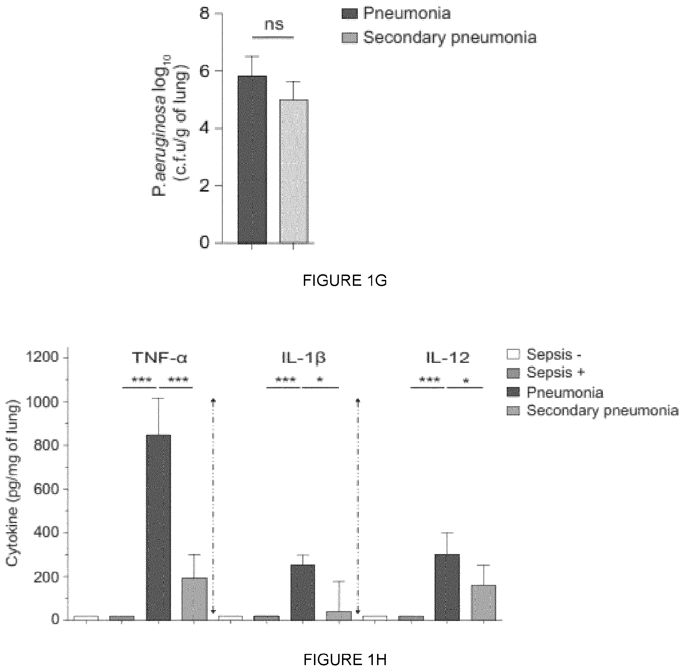

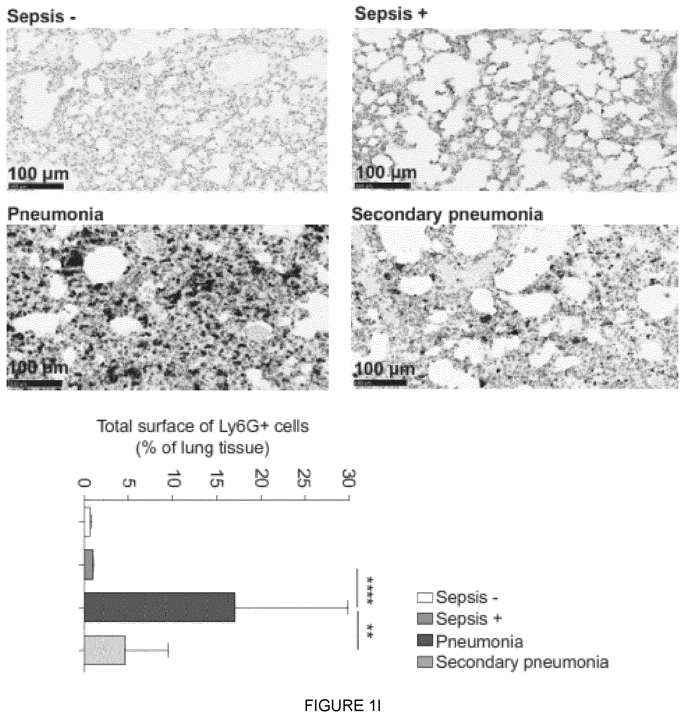

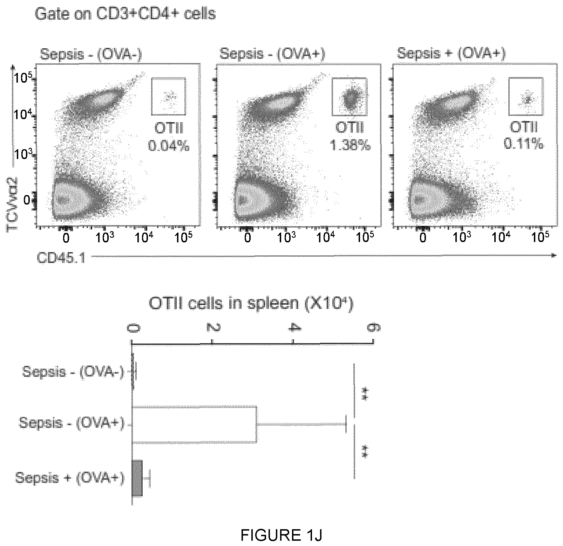

Figure 1 represents description of the primary *S*. *aureus* sepsis model that induces a susceptibility to secondary pneumonia (**A**) Experimental outline of intravenous (*i.v*) injection of *S*. *aureus* (sepsis) followed 3 days later by intra tracheal (*i.t*) injection of *P*. *aeruginosa* (pneumonia). Several groups were compared: uninfected mice (sepsis-); after 3 days of *S*. *aureus* sepsis alone (sepsis+); *P. aeruginosa* pneumonia alone (pneumonia); pneumonia after 3 days of *S*. *aureus* sepsis (secondary pneumonia). Samples were withdrawn 72 hours after sepsis or 24 hours after pneumonia unless otherwise stated. (**B**) Numeration of colony-forming units (CFU) per gram of spleen (CFU/g) and time course of the bacterial load after S. aureus sepsis. (**C**) Time course of TNF-α concentration in spleen homogenates in uninfected (sepsis-) group or 2, 6, 24 or 72 hours afeter S. aureus infection (sepsis+). (**D**) Membrane expression of CD86 (left panel) in splenic dendritic cells (DC) from mice not infected with S. aureus after 1 days (sepsis+) and membrane expression of CD69 (right panel) in splenic CD4+ T cells from mice no infected (sepsis-) or in mice infected with S. aureus after 1 days (sepsis+). (**E**) IL-1β concentrations in spleen homogenates in non-infected (sepsis-) or 24 and 72 hours after S. aureus infection (sepsis+). (**F**) Time course of mouse weight loss (% of initial weight) during pneumonia and secondary pneumonia compared to uninfected (sepsis-), septic (sepsis+) groups. (**G**) Numeration of colony-forming units (CFU) per gram of lung (CFU/g) of P. aeruginosa 24 hours after pneumonia alone (pneumonia) or secondary pneumonia. (**H**) TNF-α, IL-1β and IL-12 concentrations in lung homogenates. TNF-α was measured 6 hours after the onset of pneumonia; IL-1β and IL-12, 24 hours after pneumonia alone or secondary pneumonia. For control, TNF-α, IL-1β and IL-12 were also measured 72 hours after sepsis (sepsis+) and in uninfected (sepsis-) mice. (**I**) Lung sections were analyzed to quantify neutrophil infiltration (by anti-Ly6-G immunohistochemistry) from uninfected mice (sepsis-), 72 hours after sepsis (sepsis+), 24 hours after pneumonia alone (pneumonia) and secondary pneumonia. (**J**) Uninfected (sepsis-OVA+) or sepsis (sepsis+OVA+) CD45.2+ mice were i.v. injected with ovalbumin (OVA) and naïve OT-II cells (CD3+CD4+CD45.1+ TCRVα2+). For control, uninfected mice were injected with naïve OT-II cells (i.v) without ovalbumin (sepsis-OVA-). OT-II cells proliferation was assessed in the spleen 5 days after injection of OVA. **P < 0.05,* ***P < 0.01,* ****P* < *0.001.* Data are representative of at least two independent experiments (**B-J**) with at least five mice per group. Graphs represent median ± SD.

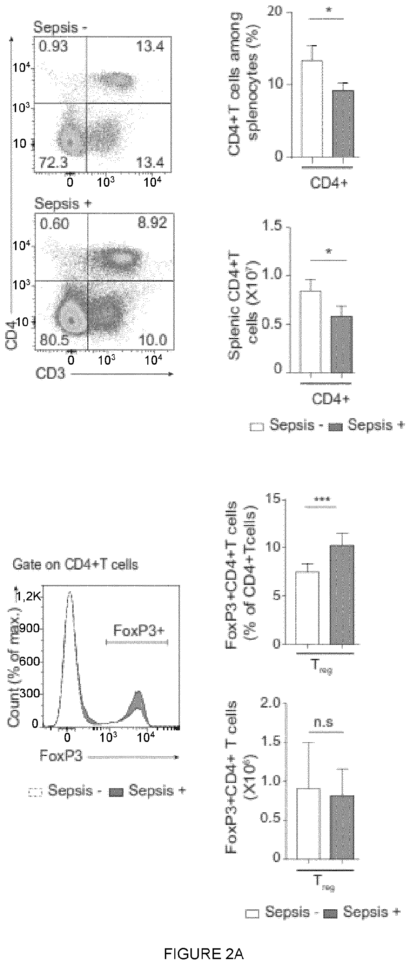

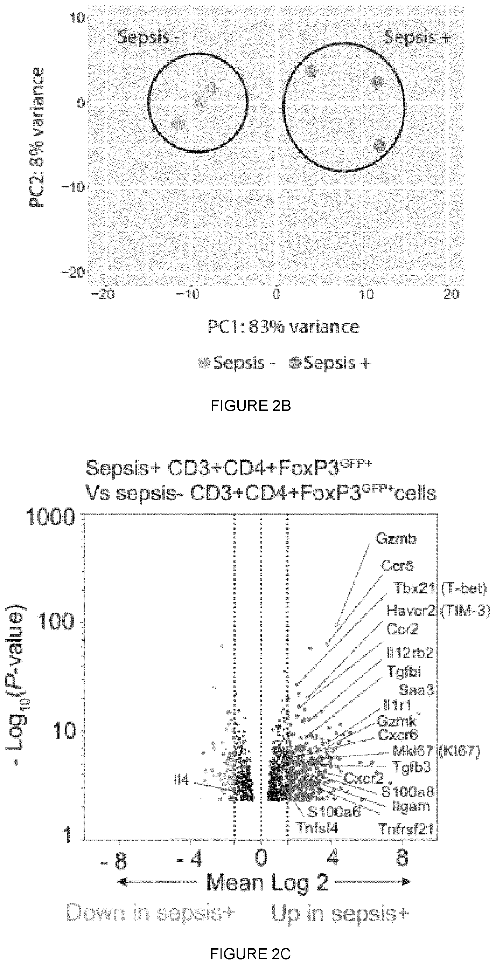

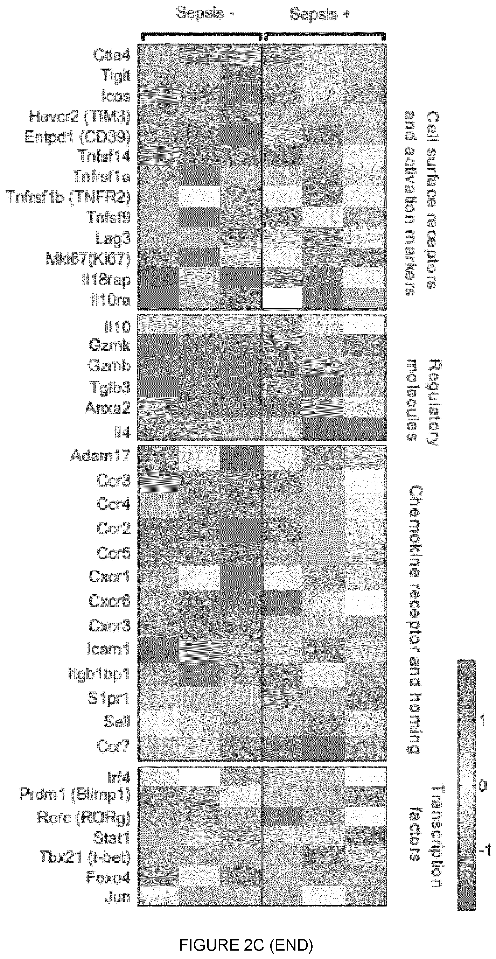

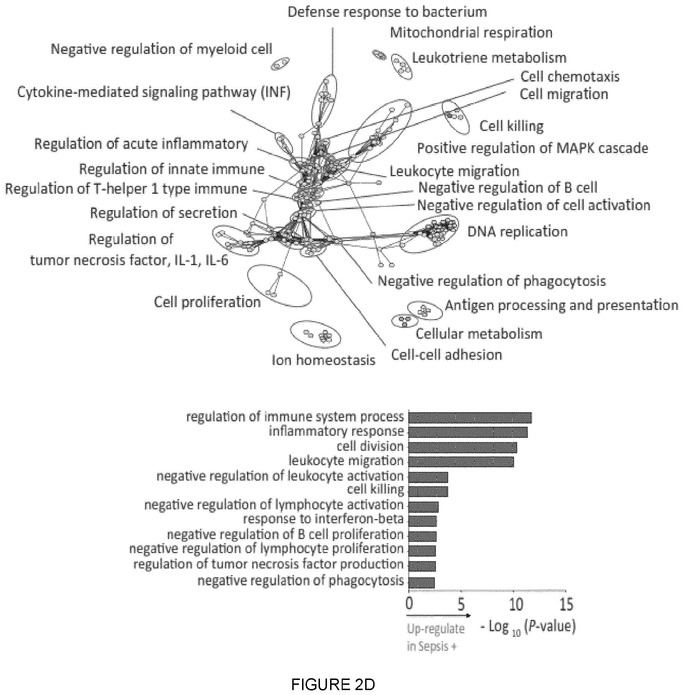

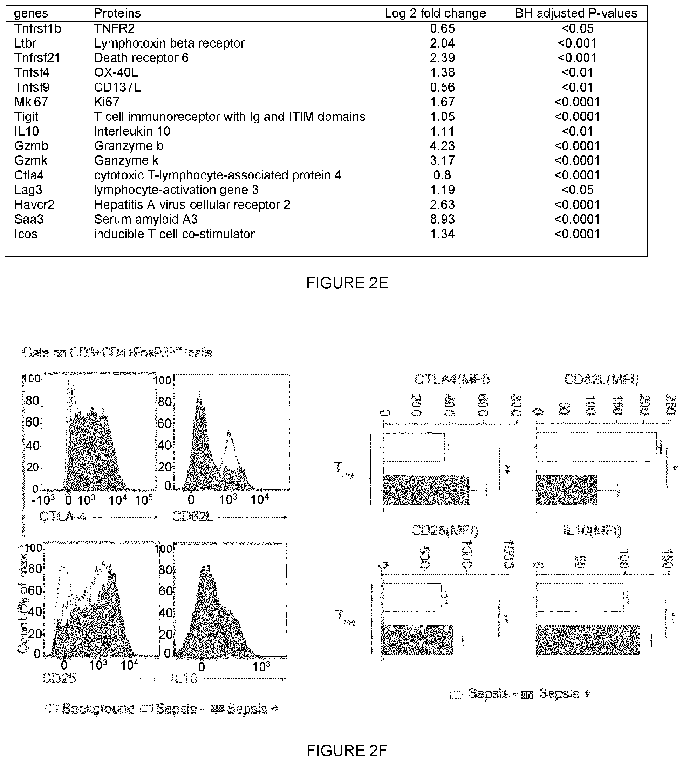

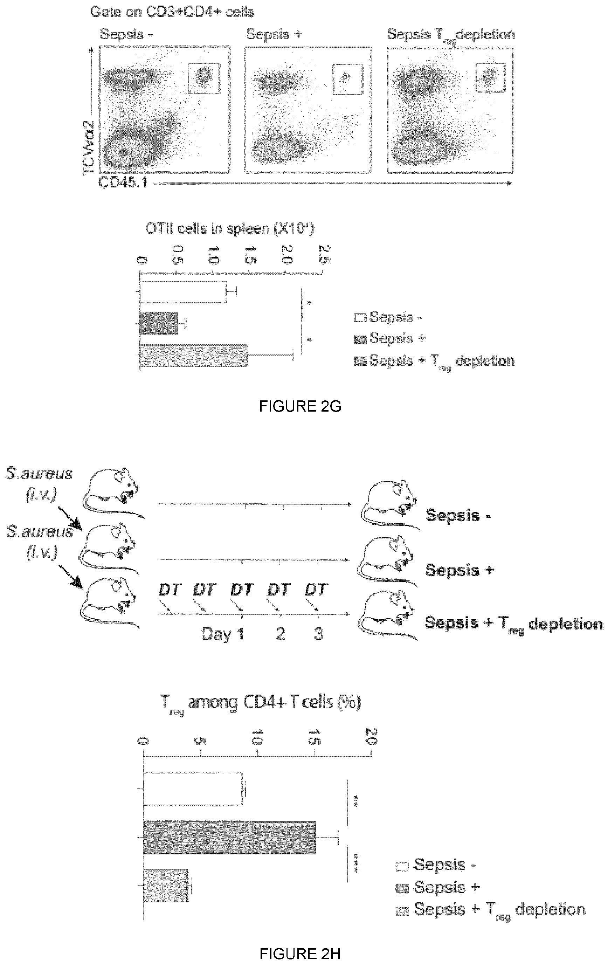

Figure 2 represents analysis of the role play by Treg in functional alterations of conventional CD4+ T cells during sepsis (**A**) Splenic CD4+ T cell proportions and numbers in uninfected (sepsis-) or septic mice (3 days after *S*. *aureus* infection, sepsis+) (first panel). Splenic Treg (CD3+CD4+FoxP3+) cells proportions and numbers in uninfected (sepsis-) or septic mice (sepsis+) (second panel). (**B**) Principal component analysis of sepsis- Treg (light grey) and sepsis+ T reg cells (dark grey). PC1 and PC2 show the percentage of variance explained for 16,112 expressed genes. (**C**) Volcano plot of differential gene expression between Treg cells from uninfected (sepsis-) and septic (sepsis⁺) mice. Significantly differentially expressed genes (*Q*-value<0.05), Up- and down-regulated genes (log2-fold change >1.5 and <-1.5) in sepsis Treg cells are highlighted in dark grey and light grey, respectively (first panel). Heatmap of differentially expressed genes (*Q*-value<0.05) encoding selected activation markers, regulatory molecules, chemokine receptors and transcription factors in Sepsis+ and Sepsis- Treg cells (second panel). (**D**) Network visualization of canonical pathway analysis based on up-regulated expressed genes (Q-value<0.05 and >1.5 log2 fold change) in sepsis Treg cells versus uninfected Treg cells (upper panel). Gene Ontology analysis (Toppgene) of up-regulated genes (corrected Q-value <0.05, >1.5 fold) in Treg cells from septic (sepsis+) versus uninfected mice (sepsis-). Up-regulated genes in sepsis Treg cells are represented highlighted in dark grey and/or circled. Bar chart displays -log10 P-value (lower panel). (**E**) TNF receptor superfamily and activation markers genes in sepsis Treg cells. (**F**) Intracellular expression of CTLA-4, IL-10 and membrane expression of CD62L, CD25 in Treg cells from uninfected (sepsis-) or septic mice (sepsis+). (**G**) Uninfected (sepsis-) or sepsis (sepsis+) CD45.2+ mice with and without DT-treated FoxP3-DTR (sepsis+ Treg depletion) were i.v injected with OVA and naive OT-II cells (CD3+CD4+CD45.1+TCRva+). OT-II proliferation was assessed 5 days later in the spleen. For Treg cells depletion mice were injected (i.p) with DT 0.5 µg for the first injection, then 0.2 µg for the following injections, administered + 12 hours, day+1, +2, +3, +5 and day+7 after *S*. *aureus* sepsis. (H) (1) Schematic diagram of the experimental outline of Treg cells depletion by Diphteria toxin (DT), (DT 0.5 µg i.p. for the first injection, then 0.2 µg i.p. for the following injections, administered +12H, day +1, +2, +3 after S. aureus sepsis). (2) Frequency of Treg cells among CD4+ T cells in spleen in uninfected mice (sepsis-), after 72H of *S*. *aureus* sepsis (sepsis+) and after 72H of *S*. *aureus* sepsis with Treg cells depletion by Diphteria toxin in DEREG mice (sepsis+Treg deletion).
   **P < 0.05,* ***P < 0.01,* ****P < 0.001.* Data are representative of at least three independent experiments with at least five mice per group (**A**, **F**, **G**; median ± SD). Data are representative of two independent experiments with at least five mice per group (**B**, **D**, **H**; median ± SD). Data are representative of one experiment with n = 2 conditions in triplicate (sepsis-, sepsis+) and 2 mice were pooled for each condition (**C**).

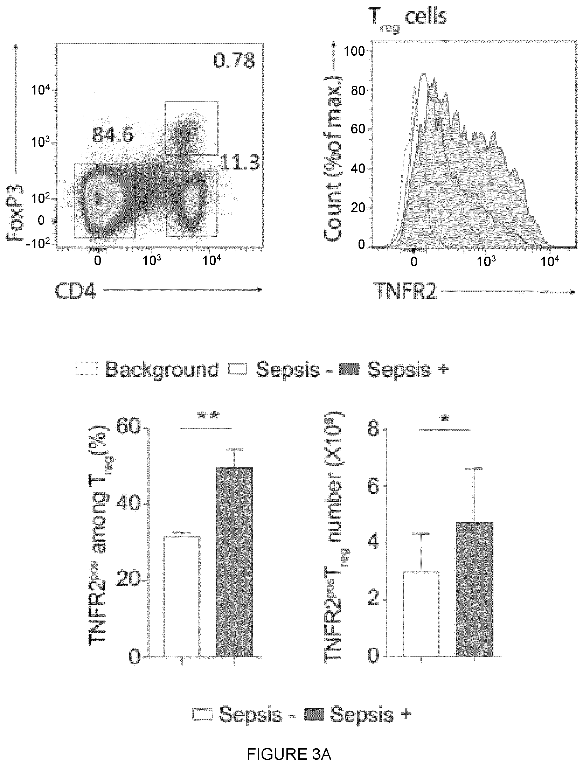

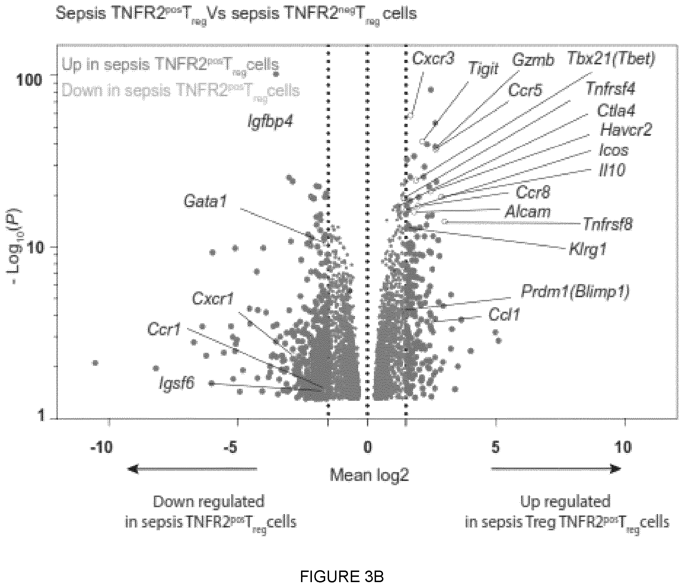

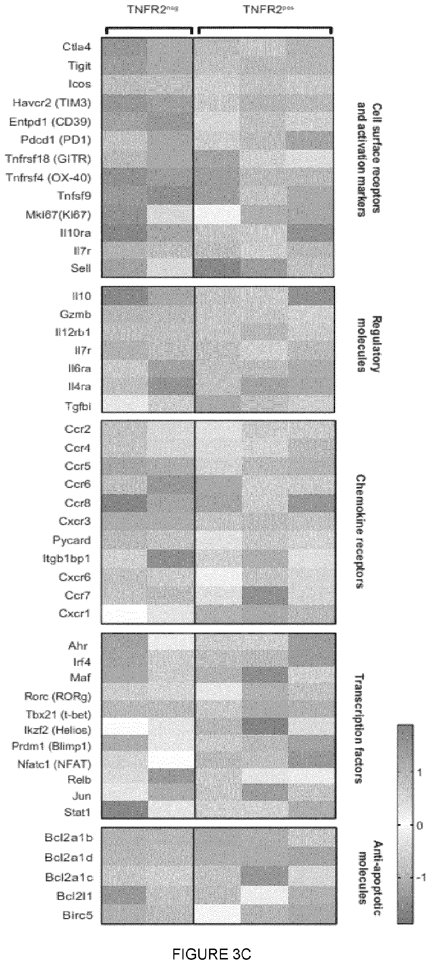

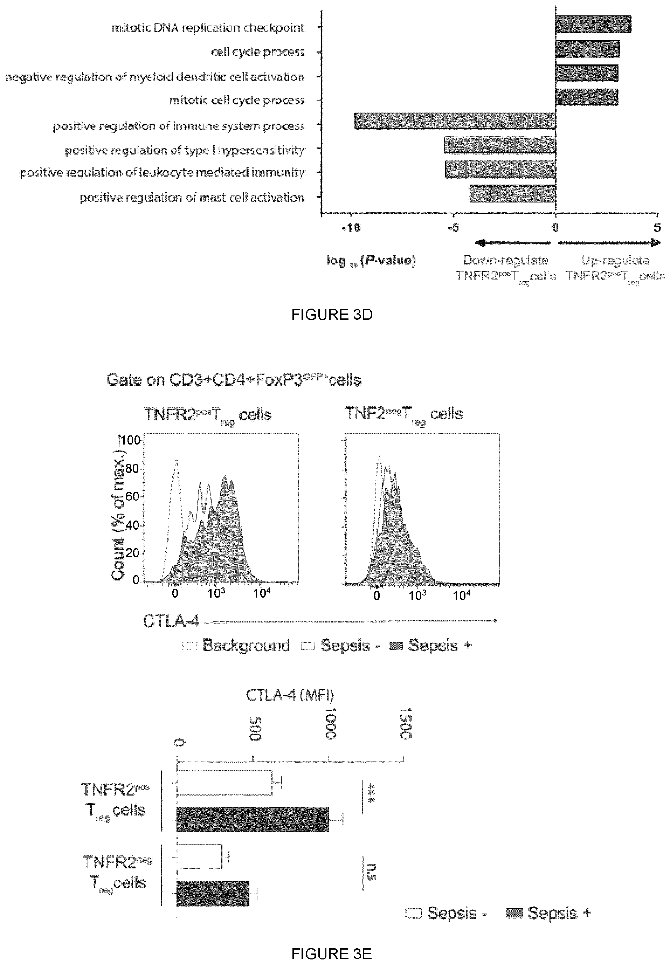

Figure 3 represents analysis of the transcriptome and phenotype of TNFR2^{neg} and TNFR2pos Tregcells during sepsis (**A**) Splenic TNFR2posTreg cells proportions and numbers in uninfected mice (sepsis-) or 3 days after *S*. *aureus* infection (sepsis+). (**B**) Volcano plot of differential gene expression between TNFR2pos Treg and TNFR2negTreg cells from septic mice. Significantly differentially expressed genes (*Q*-value<0.05), Up- and down-regulated genes (log2-fold change >1.5 and <-1.5) in sepsis TNFR2pos Treg cells are highlighted in dark grey or light grey, respectively. (**C**) Heatmap of differentially expressed genes (*Q*-value<0.05) encoding selected activation markers, regulatory molecules, chemokine receptors, transcription factors and anti-apoptotic molecules in TNFR2pos Treg and TNFR2neg Treg cells from septic mice. (**D**) Gene Ontology analysis (Toppgene) of differentially regulated genes (corrected Q-value <0.05, >1.5 fold) in TNFR2pos Treg cells in septic mice. Up- and down-regulated functions in TNFR2pos Treg cells are highlighted in dark grey and light grey. Bar chart displays -log10 P-value. (**E**) Intracellular expression of CTLA-4 in TNFR2pos Treg and TNFR2neg Treg cells from uninfected (sepsis-) or septic mice (sepsis+).
   **P* < 0.05, ***P* < 0.01, ****P* < 0.001. Data are representative of at least three independent experiments (**A**, **E**) with at least five mice per group. Graphs represent median ± SD. Data are representative of one experiment with n = 3 samples per group and 3 mice per sample (**B-D**).

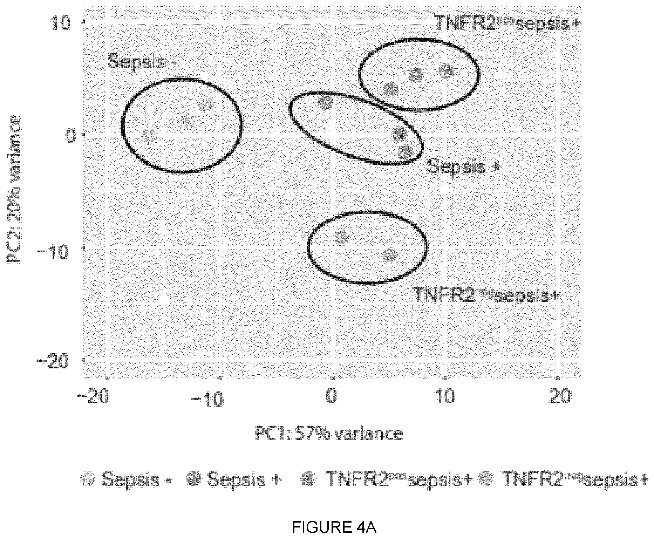

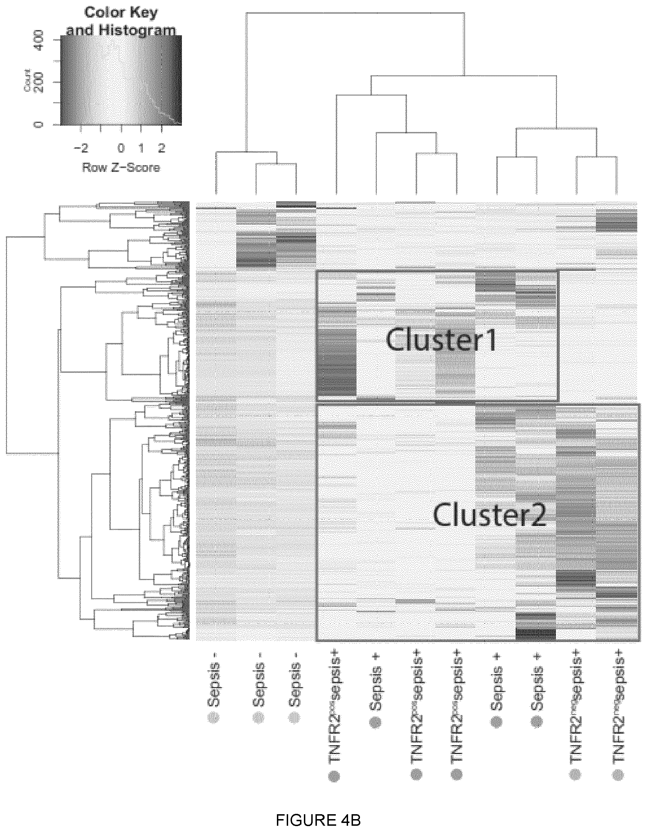

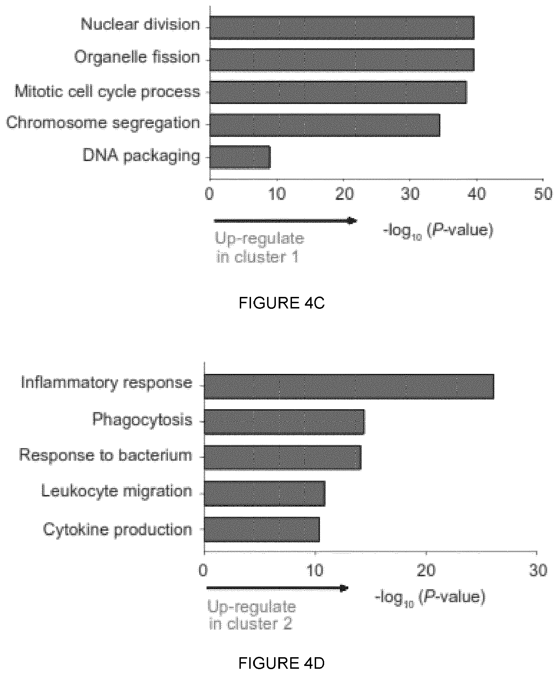

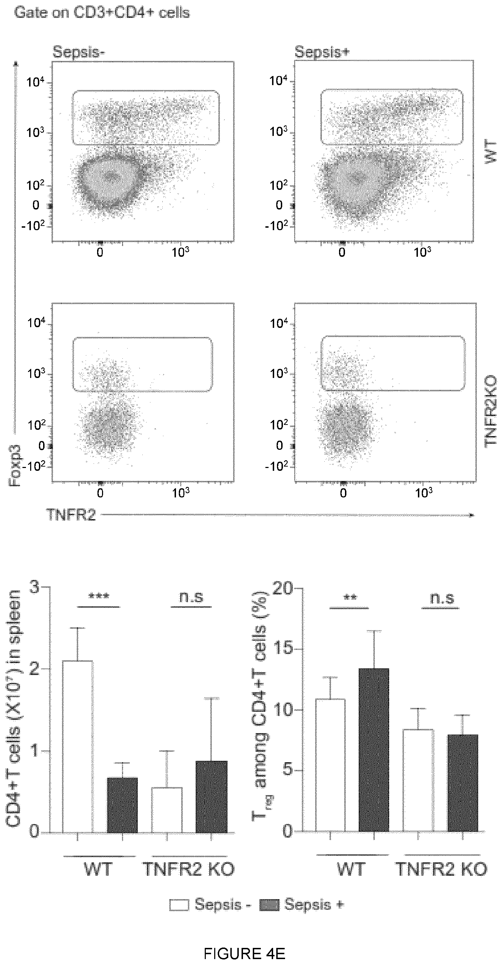

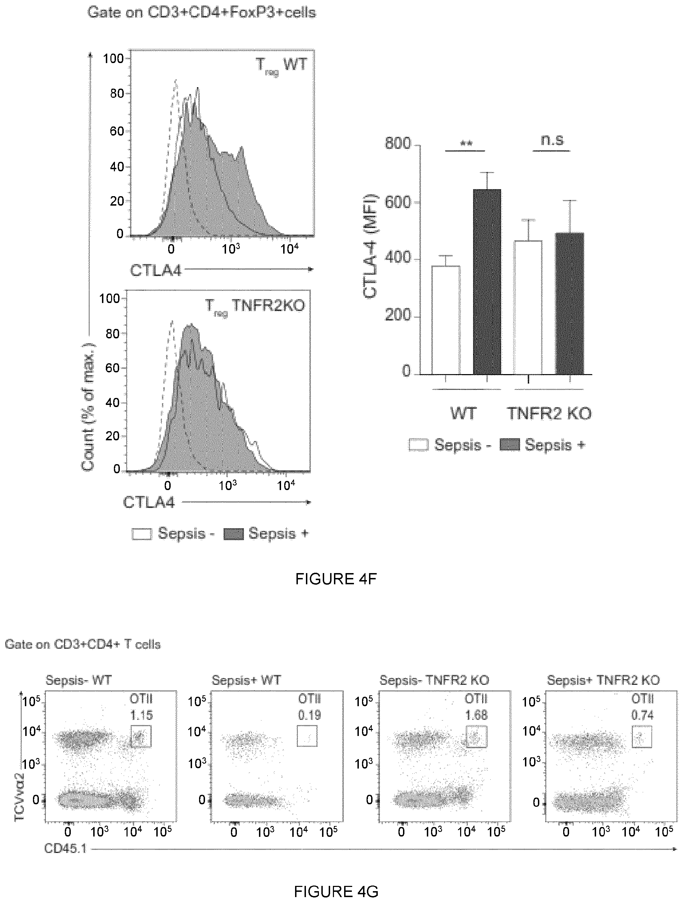

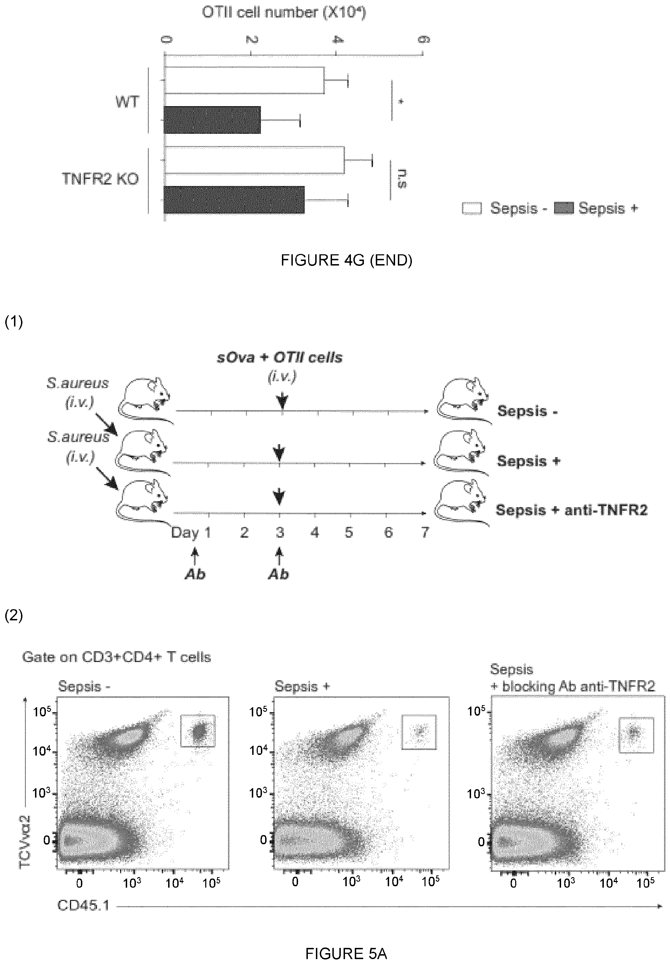

Figure 4 represents association of TNFR2pos Tregsubset to cell cycle and replication during sepsis (**A**) Principal component analysis of Treg sepsis-, Treg sepsis+, TNFR2pos Treg and TNFR2neg Treg cells. PC1 and PC2 show the percentage of variance explained for 15,337 expressed genes. (**B**) Heatmap of unsupervised hierarchical clustering of the differentially expressed gene (corrected Q-value <0.05, >1.5 fold) between sepsis+ Treg, sepsis- Treg cells. Clustering grouped the splenic Treg cells into 2 gene clusters. (**C**) Gene Ontology analysis (Toppgene) of genes in cluster 1. Bar chart displays -log 10 P-value. (**D**) Gene Ontology analysis (Toppgene) of genes in cluster 2. Bar chart displays - log10 P-value. (**E**) Splenic CD4+ T cells and Treg cells proportions and numbers from mice infected (sepsis+) or not (sepsis -) in wild type (WT) or TNFR2KO mice. ***P* < 0.01, ****P*< 0.001. Data are representative of two independent experiments with at least five mice per group. Graphs represent median ±SD. (**F**) Intracellular expression of CTLA-4 in Treg cells was measured 536 from uninfected or sepsis WT mice and in TNFR2 KO mice. (**G**) In CD45.2pos mice, uninfected (sepsis-) or septic (sepsis+) WT or TNFR2KO animals were injected with OVA (i.v) and OTII cells (CD3+CD4+CD45.1+ OT-II cell) (i.v). OT-II proliferation was assessed 5 days later in the spleen. **P* < 0.05, ***P* < 0.01, ****P<* 0.001. Data are representative of at least three independent experiments (**F**, **G**) with at least five mice per group. Data are representative of two independent experiments (**C-E**) with at least five mice per group. Graphs represent median ± SD. Data are representative of one experiment with n = 3 samples per group and 3 mice per sample (**A, B**).

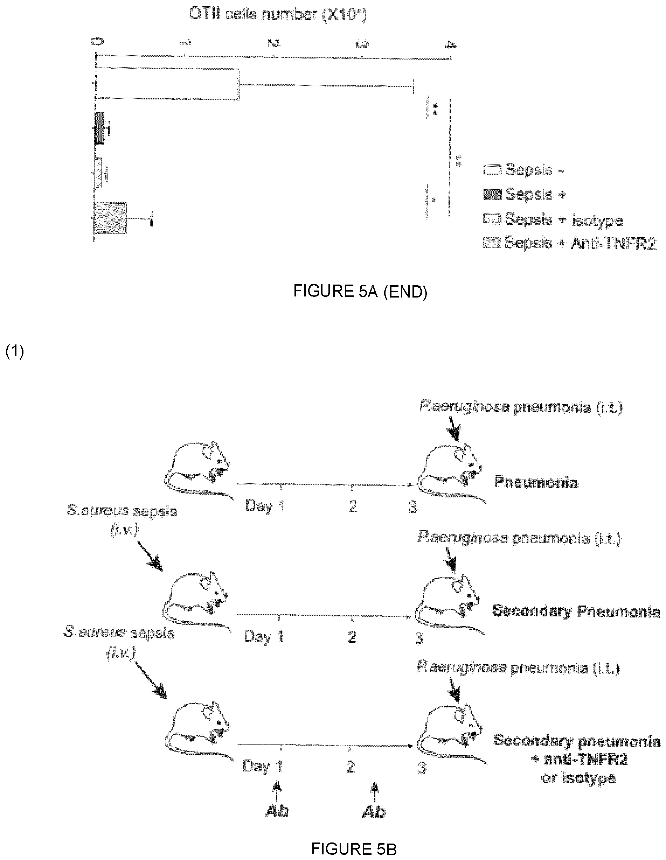

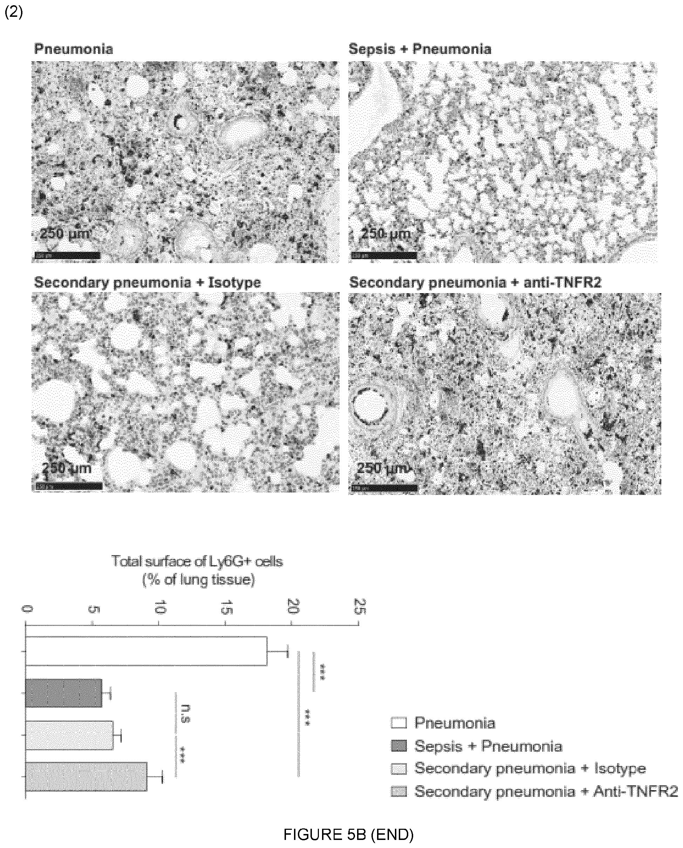

Figure 5 represents TNFR2 functions during sepsis (**A**) (1) Schematic diagram of the experimental outline of monoclonal anti-TNFR2 blocking antibody treatment and transfer of OT-II cells during *S*. *aureus* sepsis. Septic mice were treated with blocking mAb anti-TNFR2 (Sepsis+ anti-TNFR2) or with isotype control at day 1 and day 3, then mice were i.v. injected with OVA and OT-II cells. (2) The proliferation of OT-II cells was assessed 5 days later in the spleen. (**B**) (1) Schematic diagram of the experimental outline of *P*. *aeruginosa* pneumonia alone (pneumonia group), of *P*. *aeruginosa pneumonia* induced 3 days after S. *aureus* sepsis (secondary pneumonia group) treated with blocking mAb anti-TNFR2 (Sepsis+ anti-TNFR2) or with isotype control at day 1 and day 3. (2) Polynuclear infiltration was measured on lung sections collected 24 hours after *P. aeruginosa* pneumonia by anti-Ly6-G immunohistochemistry. **P*< 0.05, ***P <* 0.01, ****P*< 0.001. Data are representative of two independent experiments with at least five mice per group. Graphs represent median ± SD.

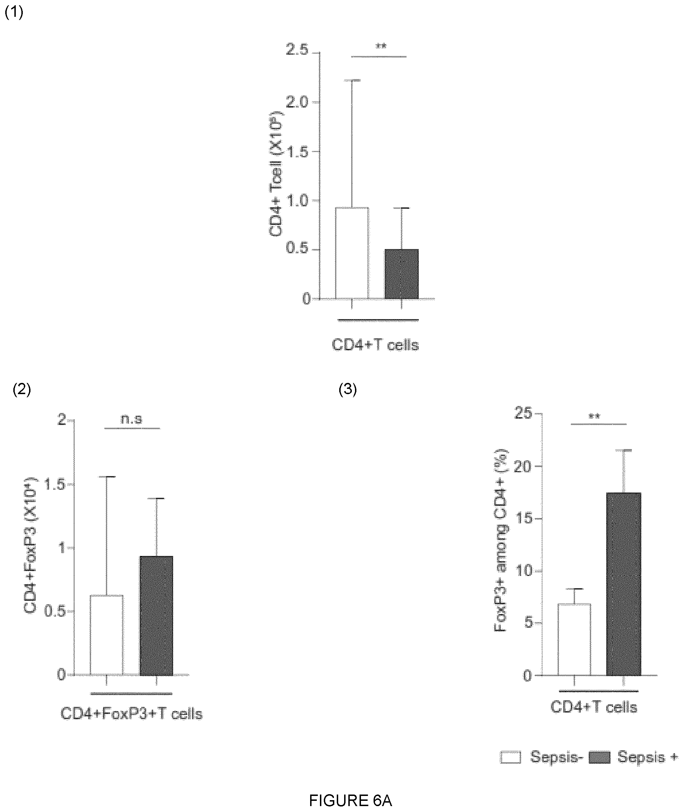

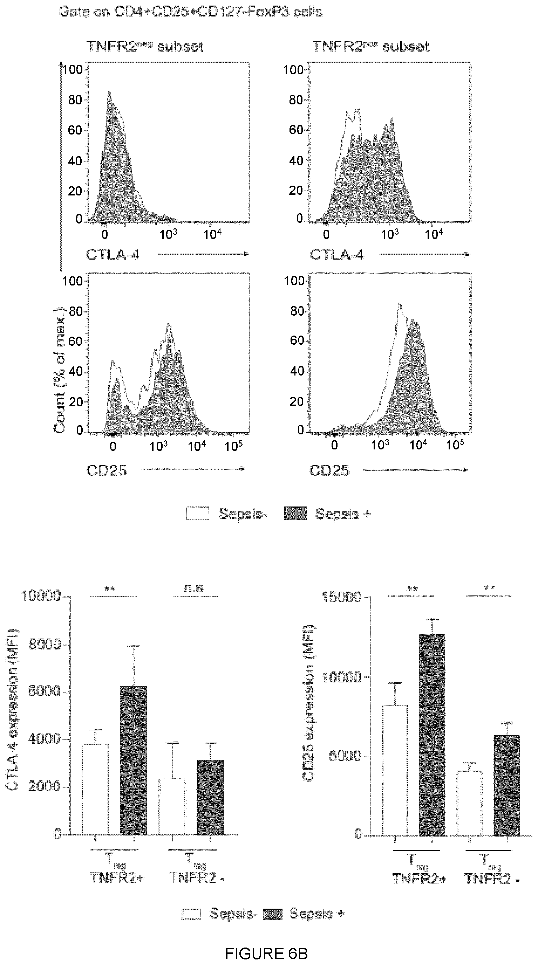

Figure 6 represents expression of TNFR2 on Treg cells in human cells (**A**) Number of CD4+ T cells (1); number (2) and frequency (3) of FoxP3+CD4+ T cells in PBMC from healthy controls cultured without (sepsis-) or with *S*. *aureus* (sepsis+). (**B**) Expression of CTLA-4, CD25 among Treg (CD4+CD25+CD127-FoxP3+) TFNR2pos or TNFR2neg cells in PBMC from healthy controls cultured without (sepsis-) or with *S*. *aureus* (sepsis+). **P* < 0.05, ***P* < 0.01, ****P* < 0.001. Data are representative of 10 healthy controls (**A-B**); Graphs represent median ± SD.

### EXAMPLES

### EXAMPLE 1 : MATERIAL & METHODS

### Mice

Mice RjOrl:SWISS and C57BL/6NRj were purchased from Janvier Laboratories (Le Genest Saint Isle, France). C57BL/6-Tg (FoxP3-DTR/EGFP)23.2Spar/Mmjax (later named DEREG) were purchased from Charles River laboratories (Saint Germain Nuelles, France), C57BL/6 Tnfrsf1btm1Mwm (later named TNFR2KO) by Dr Salomon. OT-II.Ly5.1.FoxP3EGFP mice (provided by Cedric Louvet) were obtained by crossing OVA-specfic TCR-transgenic OT-II mice (B6.Cg-Tg(TcraTcrb)425Cbn/J) [17] with Ly5.1 mice (B6.SJL-Ptprca Pepcb/BoyCrl) (from Charles River, France) and FoxP3EGFP reporter mice (from Bernard Malissen [18]). Mice were maintained in specific pathogen-free conditions at the IRS2 Institute Animal Care Facility (Nantes, France) following institutional guidelines and were used for experiments between six and fourteen weeks of age. Experimental procedures were approved by the Animal Ethics Committee of the University of Nantes (protocol #2126-2130).

### Sepsis model

*Staphylococcus aureus* ATCC29213 and *Pseudomonas aeruginosa* PAO1 strains were grown in Brain Heart Infusion broth overnight at 37°C under agitation. Immediately before use, the bacterial pellet was washed twice with PBS and the inoculum was calibrated by nephelometry.

After general anesthesia by continuous inhalation of isoflurane (3% fresh gas flow 0.8L / min), the mouse was placed in prone position and 200µL of *S*. *aureus* suspension (at 10⁹ colony-forming units (CFU)/mL) was intravenously injected through the suborbital venous plexus. When mentioned, pneumonia was induced as previously described [17]. Briefly, mice were anesthetized with isoflurane (3% fresh gas flow 0.8L / min) and placed in dorsal recumbency. Intratracheal insertion of a 24-gauge feeding needle was used to inject 75 µL of *Pseudomonas aeruginosa* suspension adjusted to 10⁸ CFU/mL (7.5 x 106 CFU/mouse).

### Murine Treg cells Isolation, Analysis

Murine splenocytes were stained with monoclonal antibodies to: anti-CD3 (145-2C11, BD Bioscience), anti-CD4 (RM4-5, BD Pharmingen), anti-CD25 (PC61, BD Horizon), anti-CD62L (MEL-14; BD Horizon), anti-TNFR2 (TR75-89, BD Pharmingen), anti-CD45.1 (A20.1; eBioscience), anti-TCR VαD (B20.1; BD Bioscience), anti-FoxP3 (MF14, Biolegend), anti-CTLA-4 (UV10-4B9, Biolegend), anti-IL10 (JES5-16E3; BD Horizon), Fixable Viability Dye (eBioscience). IL-10 staining was performed using the Fixation/permeabilization solution for cytokines (Fixation/Permeabilization Solution Kit with BD GolgiPlug; Biosciences) and FoxP3, CTLA-4 straining using the FoxP3 Transcription Factor Staining Buffer Kit (eBioscience) transcription factors, according to manufacturer instructions. Samples were acquired on LSR-II (Becton Dickinson) and analyzed using Flowjo Software (TreeStar Inc, Ashland, OR).

### Induction of OTII cell proliferation after adoptive transfer

OT-II T cells were purified from pooled lymph nodes and spleen of OT-II mice after lysis of the red blood cells and CD4+T enrichment (Miltenyi Biotech, Germany) (purity 85-95%). Mice were injected i.v. with soluble OVA (0.1 mg, A5503, Sigma-Aldrich, France) and OT-II cells (0.5 - 1.5 × 10⁶ cells). Five days later, cells from the spleen were stained with anti-CD4, CD45.1, anti-TCRVa2, and resuspended in buffer containing 1-3 × 10⁴ blank calibration particles (Becton Dickinson). The total number of live dividing OT-II was calculated from the number of dividing cells relative to the number of beads present in each sample. 132 Control mice receiving OT-II cells with PBS alone (without OVA).

### Cytokine levels, bacteriological counts and histology and immunohistochemistry analysis

Cytokines levels in the lungs and spleens were measured by ELISA according to manufacturer instructions (eBioscience, France), bacterial counts, histology and immunohistochemistry analysis were determined as previously described on spleen and lung [17].

### Treg Cell Depletion

Diphtheria toxin (0.5 µg i.p for the first injection, then 0.2 µg i.p for the following injection - Sigma-Aldrich, France) was administrated to FoxP3GFP-DTR (DEREG) mice (timing of injections are detailed in the figure legends).

### Anti-TNFR2 monoclonal antibody treatments

Anti-TNFR2 antibodies (TR75-54.7, isotype Rat IgG2b, κ, 500 µg/mice, Bioxcell) were intravenously administered 15 min before infection, then 15 min before injection of the OT-II T lymphocytes and OVALBUMIN or secondary pneumonia, 3 days after S. aureus sepsis.

### Human PBMC sepsis induction and Treg cells analysis

Peripheral blood mononuclear cell (PBMC) samples were collected from healthy blood donors recruited at the Blood Transfusion Center (Etablissement Français du Sang, Nantes, France). PBMCs were isolated by density gradient, frozen in liquid nitrogen in a 10% DMSO solution and stored until analysis. After 2 hrs of *in vitro* co-culture of PBMC (1 × 10⁶ cells) with bacterial suspension (S. *aureus*), PBMC were treated with gentamicin for 20 min. After suspension in a buffer solution (PBS EDTA 0.1% BSA 0.5%) and centrifugation (1500 rpm, 10 min at room temperature) the cell pellet was suspended in PBS and 154 FACS analyzed 24 hrs later. Treg cells were identified with anti-CD4 (REA623, Miltenyi), anti-CD25 (REA 945, Miltenyi) and anti-CD127 (REA614, Miltenyi), anti-CD120b (REA520, Miltenyi), anti-CTLA-4 (REA1003, Miltenyi), anti-FoxP3 (3G3, Miltenyi). Treg cells were identified as CD4+CD25highCD127low/-FoxP3+.

### RNA-seq

For cell analysis of RNA, Treg (CD4+FoxP3 GFP+) cells sorted with FACSAria III (purity > 95%) and obtained from pooled spleen (2 mice per sample for Treg cells from uninfected and sepsis mice, or 3 mice for TNFR2pos and TNFR2neg Treg cells from sepsis mice). Treg cells were lysed for RNA preparation using the QIAGEN RNEasy plus mini-kit. Three independent RNA samples were obtained from sepsis Treg cells, uninfected TNFR2pos Tregand TNFR2neg Treg cells. One of the TNFR samples was removed as it showed as an outlier (z-score of total expressed genes > -1.5).

cDNA libraries, RNA sequencing Library preparation and Illumina sequencing were performed at the Ecole normale superieure genomic core facility (Paris, France). 10 ng of total RNA were amplified and converted to cDNA using SMART-Seq v4 Ultra Low Input RNA kit (Clontech). Afterwards an average of 150 pg of amplified cDNA was used to prepare library following Nextera XT DNA kit (Illumina). Libraries were multiplexed 23 by 12 on 4 high-output flow cells. A 75 bp read sequencing was performed on a NextSeq 500 device (Illumina). A mean of 91,656,036± 218,376 passing Illumina quality filter reads was obtained for each of the 12 samples.

The analyses were performed using the Eoulsan pipeline(1), including read filtering, mapping, alignment filtering, read quantification, normalisation and differential analysis: Before mapping, poly N read tails were trimmed, reads ≤40 bases were removed, and reads with quality mean ≤30 were discarded. Reads were then aligned against the Mus musculus genome from Ensembl version 91 using STAR (version 2.5.2b)(2). Alignments from reads matching more than once on the reference genome were removed using Java version of samtools(3). To compute gene expression, Mus musculus GTF genome annotation version 91 from Ensembl database was used. All overlapping regions between alignments and referenced exons were counted using HTSeq-count 0.5.3(4). The sample counts were normalized using DESeq 2 1.8.1(5). Statistical treatments and differential analyses were also performed using DESeq2 1.8.1.

For gene set enrichment analysis (GSEA)(6,7), a ranked list of the differentially expressed genes was used with the GSEA Desktop Application v2.2.1, and the Molecular Signatures Database v45.1. Sample label (phenotype) permutation was always used and an FDRcut-off was thus applied (<0.25) (http://software.broadinstitute.org/cancer/software/gsea/wiki/index.php/Gsea)

Differential expression analyses were performed using the DESeq2 package(5). Low abundance genes were filtered out leaving 16112 genes in the sepsis analysis and 15337 genes for the TNFR DEG analysis. Differential expressed genes were additionally filtered by an absolute log2 Fold-change of 1.5while controlling the false discovery rate with the Benjamini-Hochberg method (5%). Gene ontology analysis was carried out using Toppgene suite(8). Adjusted-p-values according to BH method are noted as Q-values through the paper in the RNA-seq analysis.

### Statistical Analysis

Data were plotted using GraphPad prism (San Diego. United States). Mann-Whitney unpaired test was used for comparisons between 2 groups. A Kruskall-Wallis test was used for multiple comparisons. When the difference was significant, a Fisher test was used for inter-group comparison. P-value < 0.05 for statistical significance.

### EXAMPLE 2: RESULTS

### Sepsis increases the susceptibility to secondary pneumonia and induces immunosuppression

To mimic the frequent clinical scenario of sepsis followed by secondary pneumonia [4], sepsis was induced in mice with an intravenous injection of *Staphylococcus aureus (S. aureus)* followed 3 days later by intra-tracheal injection of *Pseudomonas aeruginosa (P. aeruginosa)* to induce secondary pneumonia (**Fig. 1A**). In mice with sepsis alone, a spontaneous bacterial clearance (**Fig. 1B**), and a systemic inflammatory response in the spleen (**Fig**. **1C-E**) were observed, as reported in septic patients [1,18]. In order to find-out if sepsis alters the lung response to a secondary pneumonia, the host response was compared to a secondary *P*. *aeruginosa* pneumonia in mice previously infected or not by *S*. *aureus.* A dramatic weight loss was observed until day 6 during secondary pneumonia that could not be explained by a higher burden of bacteria or by the additive effect of the two hits taken separately [19,20] (**Fig. 1F-G**). The inflammatory response was also impaired during secondary pneumonia compared with pneumonia alone (**Fig. 1H-I**). These results prompted to evaluate if an alteration of the CD4+ T cells compartment induced by *S*. *Aureus* sepsis could explain the impairment of the host response against the secondary pneumonia. The ability of the immune system to respond *in vivo* to a newly encountered extracellular antigen after the sepsis initiation was tested, as assessed by the proliferation of CD4+ T cells. For this purpose, CD4+ T cells from OT-II CD45.1pos transgenic mice expressing a T cell receptor which is specific for ovalbumin-derived peptide were used and OT-II cell proliferation was assessed in the spleen 5 days after adoptive transfer and ovalbumin immunization of septic mice. Strikingly, the number of OT-II cells was dramatically decreased in septic mice infected with *S*. *aureus* compared with uninfected control mice (**Fig. 1J**). It was then questioned if the 198 decreased OTII proliferation was associated with CD4+ T cells intrinsic defect. Finally, the number of splenic CD4+ T cells was also drastically decreased after *S*. *aureus* sepsis (**Fig. 2A**) as observed in patients [7]. This series of experiments demonstrated that this model of *S*. *aureus* sepsis mimicked a clinical scenario with severe direct and indirect impairment of the CD4+ T cells, *in vivo,* associated with an altered pulmonary response to secondary pneumonia.

### Sepsis induces major Treg cells activation that generates a suppressive environment

The role of Treg cells in the CD4+ T cells impairment caused by S. *aureus* sepsis was then assessed. Despite the important CD4+ T cells depletion, the Treg cell number remained unaltered, resulting in an increased percentage of Treg cells among CD4+ T cells (**Fig. 2A**), and advocating for a specific response to sepsis of Treg cells among CD4+ T cells.

In order to better characterize the effects of sepsis on Treg cells, the transcriptome of splenic Treg cells was anlyzed 3 days after the induction of sepsis (additional detail on the method for making these measurements is provided in an online data supplement). Principal component analysis (PCA) of 16,112 expressed genes revealed that gene expression profiles of uninfected Treg cells and sepsis Treg cells were separated by PC1 (81% Variance explained), suggesting that most gene expression variance was associated to infection rather than to experimental variation (PC2 8%) (**Fig. 2B**). Thus, differentially expressed genes was estimated, and 492 were found to be up-regulated, and 96 were found to be downregulated with an absolute log2-fold change above 1.5 (*Q*-value<0.05) (**Fig. 2C**). *Gzmb, Mki67* (Ki67), *Havcr2* (TIM-3) were among the most strongly expressed genes in sepsis Treg cells, suggesting that Treg cells from septic mice were highly activated compared with controls. To gain a more comprehensive overview of the underlying molecular pathways implicated in the sepsis, these 220 differentially expressed genes (DEG) were analyzed for gene ontology (GO) enrichment. This analysis revealed that sepsis was associated with a transcriptional programming driving biological processes, including cell cycle replication and immunological response, such as adaptive immune regulation (**Fig. 2D**).

The *Irf4* and *Prdm1* (Blimp 1), transcriptional factors which are required in a non225 redundant manner to maintain effector Treg cells differentiation and functions [21,22], were also up-regulated in sepsis Treg cells (**Fig. 2C**). Many of the up-regulated genes (**Fig. 2C** and **Fig. 2E**) encode for molecules involved in the activation and suppressive function of Treg suggesting that Treg from septic mice were highly suppressive [23]. The differential expression of two important markers of activation (CD62L, CD25) [24,25] and of two suppressive mediators (CTLA-4 and IL-10) [24] were validated at a protein level (**Fig. 2F**). Overall, these results suggest that sepsis triggered the maintenance and the activation of Treg cells.

To demonstrate the role of Treg cells in sepsis-induced immunosuppression, Treg were depleted after the initiation of *S*. *aureus* sepsis by injection of diphtheria toxin in mice expressing the Diptheria Toxin Receptor (DTR) under the control of the FoxP3 gene promotor (FoxP3DTR) mice. Importantly, the depletion of Treg cells in septic mice restored the expansion of ovalbumin-specific OT-II cells (**Fig. 2G**) and partially limited CD4+ T cell depletion in infected mice (**Fig. 2H**). All together, these data demonstrated that during sepsis, the early activation of Treg cells participates to the immunosuppression that increases the susceptibility to secondary pneumonia.

### TNFR2pos Tregcells are highly activated and may suppress CD4+ T cells during sepsis

While activation of Treg cells have been described during chronic inflammation [27], little is known regarding their early activation during the acute inflammatory response of sepsis. The expression of TNFR2 identifies a subset of Treg cells with the highest suppressive capacity in tumors [29,30], and the transcript of TNFR2 gene as well as those of its downstream NF-kB signaling pathway were increased in septic Treg (**Fig. 2C**). It was thus hypothesized that the activation of Treg cells during sepsis was dependent of TNFR2. To reinforce this hypothesis, the proportion of the TNFR2pos subset among Treg cells in septic mice was measured and it was found that the percentage and the number of TNFR2pos Treg cells were increased as compared to uninfected mice (**Fig. 3A**).

To evaluate whether there is a specific gene activation of the TNFR2pos Treg cells subset, the transcriptomic differences were investigated between splenic TNFR2pos and TNFR2neg Treg cells three days after the onset of sepsis. 194 up-regulated and 415 downregulated genes were found in TNFR2pos Tregcells (>1.5 log2-fold change, Q-value<0.05) (**Fig. 3B**). Interestingly, *Gzmb, IL10, CCR5, Havcr2* (TIM-3), Tigit genes were up-regulated in TNFR2pos Treg cells. Also, many activation markers, regulatory molecules, chemokine receptors, transcription factors and anti-apoptotic molecules were up-regulated in sepsis TNFR2pos Treg cells (**Fig. 3C**). The results of the GO analysis suggest that among Treg cells, TNFR2pos Tregcells endorse the majority of effector, immunosuppressive functions compared with TNFR2neg Treg cells (**Fig. 3D**). Finally, the preferential up-regulation of CTLA-4 in TNFR2pos Treg cells was validated by flow cytometry (**Fig. 3E**).

### TNFR2pos Treg subset was associated to cell cycle and replication during sepsis

To investigate how the TNFR2pos Treg cells contribute to the transcriptomic signature of Treg cells observed in sepsis (**Fig. 2**), the sepsis DEG was used to map each condition on a reduced dimensional space using PCA (**Fig. 4A**). During sepsis, total Treg cells were closer to TNFR2pos Treg subset than to TNFR2neg Treg cells, suggesting that the TNRF2pos Treg cells transcriptome is a major contributor of the Treg cells response. To gain a deeper understanding of the role of TNFR2 on the activation of Treg, cells, unsupervised hierarchical clustering of the differentially expressed gene of the Sepsis DEG was used in all four conditions (**Fig. 4B**) and it was found that differentially expressed genes can be subdivided in two separate clusters. The cluster 1, corresponding to the genes activated in sepsis Treg cells and the TNFR2pos subset, were associated to cell cycle and replication (**Fig. 4C**). The genes in cluster 2, corresponding to those activated in sepsis Treg cells and in the TNFR2neg subset, drive inflammatory response (**Fig. 4D**). This series of experiments strongly suggest that TNFR2pos Treg cells display a higher lineage stability, a higher capacity to proliferate and the TNFR2pos Treg cells retain the majority of suppressive functions during sepsis.

To demonstrate, *in vivo,* the role of TNFR2 in the activation of Treg cells during sepsis, TNFR2 knock-out mice (TNFR2 KO) were infected. Uninfected TNFR2 deficient mice spontaneously display a lower number of splenic CD4 +T cells than wild type mice (**Fig. 4E**). In TNFR2 deficient mice, sepsis does not increase the percentage of Treg cells among CD4+ T cells (**Fig. 4E**). The expression of CTLA4 remained unchanged during sepsis in deficient mice whereas an important increase was observed in wild type mice (**Fig. 4F**). Moreover, the expansion of OT-II cells in response to immunization with ovalbumin was impaired in wild type mice but remained unchanged in TNR2 deficient mice during sepsis (**Fig. 4G**). Overall, these results demonstrate the role of TNFR2 in the activation of Treg cells during sepsis.

### Therapeutic use of a blocking monoclonal anti TNFR2 antibody

It was then questioned if the inhibition of TNFR2 with a blocking monoclonal antibody could decrease the susceptibility to secondary pneumonia. To respond to this question, wild type mice were treated from day 1 to day 3 of sepsis by intraperitoneal injection with a blocking monoclonal antibody against TNFR2 (**Fig. 5A-B**). The treatment of septic mice with the blocking anti-TNFR2 antibody partially restored the expansion of OT-II cells in response to sOVA injected three days after the onset of sepsis (**Fig. 5A**). In septic mice treated with the blocking anti-TNFR2 antibody, the lung neutrophil infiltrate was restored during secondary pneumonia [31] (**Fig. 5B**). Overall, these series of experiments show that a monoclonal antibody blocking TNFR2 has the potential to treat sepsis-induced immunosuppression.

### Expression of TNFR2 on Treg cells in human cells

It was then aimed to verify the extrapolation of above findings in humans. Peripheral blood mononuclear cells from healthy donors cultured *in vitro* with S. *aureus* for 2 hours were analyzed. Under sepsis condition, the total CD4+ T cells number was drastically decreased and the number of CD4+FoxP3+ T cells was not altered; however, the percentage of FoxP3+ cells among CD4+ T cells was significantly enhanced (**Fig. 6A**). The expression of CTLA-4 and CD25 were preferentially up-regulated in the presence of S. *aureus* in the TNFR2pos Treg subset (**Fig. 6B**). These experimental results in human cells parallel those obtained in the mouse model.

In the results, 3 major findings suggest an enhanced Treg cells function in the septic host: 1) Enhanced Treg ratio; 2) restoration of number/proliferation of CD4+ T cells after Treg depletion and 3) transcriptomic signature of activated Treg cells confirmed at the protein level by the up-regulation of CTLA-4 expression and IL-10 production. The results can thus be summarized as follows: Treg cells are early involved in the immunosuppression observed during sepsis and increase the susceptibility to secondary pneumonia. Among the population of Treg cells, the TNFR2pos subset appears to play a particular role since a specific blockade or deletion of TNFR2 enhances the number anf functions of CD4+ T cells. The results obtained in cells from healthy donors parallel those obtained in mice. A particular focus was placed on the capacity of the immune system to face secondary bacterial pneumonia, and an original and easy to perform sepsis model was developed in which a significant decrease in the absolute number of CD4+ T cels was observed.

The restricted expression of TNFR2 on Treg cells is thus ideal for therapeutic interventions because targeting this receptor could reduce the toxicity observed with untargeted inhibition of Treg such as anti-CTLA4 [32]. We therefore propose an original and tailored therapy for sepsis patients by blocking Treg cells, for example with anti-TNFR2 antibodies, providing that stratification is necessary to treat solely septic patients with immunosuppression.

### List of references

1. Delano MJ, Ward PA. Sepsis-induced immune dysfunction: can immune therapies reduce mortality? J Clin Invest. 2016; 126(1):23-31.
2. Vught LA van, Klein Klouwenberg PMC, Spitoni C, et al. Incidence, Risk Factors, and Attributable Mortality of Secondary Infections in the Intensive Care Unit After Admission for Sepsis. JAMA. 2016; 315(14):1469-1479.
3. Hotchkiss RS, Monneret G, Payen D. Immunosuppression in sepsis: a novel understanding of the disorder and a new therapeutic approach. Lancet Infect Dis. 2013; 13(3):260-268.
4. Hotchkiss RS, Monneret G, Payen D. Sepsis-induced immunosuppression: from cellular dysfunctions to immunotherapy. Nat Rev Immunol. 2013; 13(12):862-874.
5. Abraham E, Glauser MP, Butler T, et al. p55 Tumor necrosis factor receptor fusion protein in the treatment of patients with severe sepsis and septic shock. A randomized controlled multicenter trial. Ro 45-2081 Study Group. JAMA. 1997; 277(19):1531-1538.
6. Bernard GR, Francois B, Mira J-P, et al. Evaluating the efficacy and safety of two doses of the polyclonal anti-tumor necrosis factor-a fragment antibody AZD9773 in adult patients with severe sepsis and/or septic shock: randomized, double-blind, placebo-controlled phase IIb study*. Crit Care Med. 2014; 42(3):504-511.
7. Schwulst SJ, Grayson MH, DiPasco PJ, et al. Agonistic monoclonal antibody against CD40 receptor decreases lymphocyte apoptosis and improves survival in sepsis. J Immunol Md 1950. 2006; 177(1):557-565.
8. Boomer JS, To K, Chang KC, et al. Immunosuppression in patients who die of sepsis and multiple organ failure. JAMA J Am Med Assoc. 2011; 306(23):2594-2605.
9. Drewry AM, Samra N, Skrupky LP; Fuller BM, Compton SM, Hotchkiss RS. Persistent lymphopenia after diagnosis of sepsis predicts mortality. Shock Augusta Ga. 2014; 42(5):383-391.
10. Belkaid Y, Piccirillo CA, Mendez S, Shevach EM, Sacks DL. CD4+CD25+ regulatory T cells control Leishmania major persistence and immunity. Nature. 2002; 420(6915):502-507.
11. Qureshi OS, Zheng Y, Nakamura K, et al. Trans-endocytosis of CD80 and CD86: a molecular basis for the cell-extrinsic function of CTLA-4. Science. 2011; 332(6029) :600-603.
12. Roquilly A, McWilliam HEG, Jacqueline C, et al. Local Modulation of Antigen-Presenting Cell Development after Resolution of Pneumonia Induces Long-Term Susceptibility to Secondary Infections. Immunity. 2017; 47(1):135-147.e5.
13. Chen X, Subleski JJ, Kopf H, Howard OMZ, Männel DN, Oppenheim JJ. Cutting edge: expression of TNFR2 defines a maximally suppressive subset of mouse CD4+CD25+FoxP3+ T regulatory cells: applicability to tumor-infiltrating T regulatory cells. J Immunol Baltim Md 1950. 2008; 180(10):6467-6471.
14. Chopra M, Riedel SS, Biehl M, et al. Tumor necrosis factor receptor 2-dependent homeostasis of regulatory T cells as a player in TNF-induced experimental metastasis. Carcinogenesis. 2013; 34(6): 1296-1303.
15. Hotchkiss RS, Nat Rev Dis Primers. 2016 Jun 30;2:16045
16. Venet, Monneret Nat Rev Nephrol. 2018 Feb;14(2):121-137. doi: 10.1038/nrneph.2017.165. Epub **2017** Dec 11.
17. Barnden MJ, Allison J, Heath WR, Carbone FR. Defective TCR expression in transgenic mice constructed using cDNA-based alpha- and beta-chain genes under the control of heterologous regulatory elements. Immunol Cell Biol. 1998; 76(1):34-40.
18. Wang Y, Kissenpfennig A, Mingueneau M, et al. Th2 lymphoproliferative disorder of LatY136F mutant mice unfolds independently of TCR-MHC engagement and is insensitive to the action of Foxp3+ regulatory T cells. J Immunol Baltim Md 1950. 2008; 180(3):1565-1575.
19. Broquet A, Roquilly A, Jacqueline C, Potel G, Caillon J, Asehnoune K. Depletion of natural killer cells increases mice susceptibility in a Pseudomonas aeruginosa pneumonia model. Crit Care Med. 2014; 42(6):e441-450.
20. Shankar-Hari M, Phillips GS, Levy ML, et al. Developing a New Definition and Assessing New Clinical Criteria for Septic Shock: For the Third International Consensus Definitions for Sepsis and Septic Shock (Sepsis-3). JAMA. 2016; 315(8):775-787.
21. Delano MJ, Moldawer LL. The origins of cachexia in acute and chronic inflammatory diseases. Nutr Clin Pract Off Publ Am Soc Parenter Enter Nutr. 2006; 21(1):68-81.
22. Pugh AM, Auteri NJ, Goetzman HS, Caldwell CC, Nomellini V. A Murine Model of Persistent Inflammation, Immune Suppression, and Catabolism Syndrome. Int J Mol Sci. 2017; 18(8).
23. Cretney E, Xin A, Shi W, et al. The transcription factors Blimp-1 and IRF4 jointly control the differentiation and function of effector regulatory T cells. Nat Immunol. 2011; 12(4):304-311.
24. Vasanthakumar A, Liao Y, Teh P, et al. The TNF Receptor Superfamily-NF-κB Axis Is Critical to Maintain Effector Regulatory T Cells in Lymphoid and Non-lymphoid Tissues. Cell Rep. 2017; 20(12):2906-2920.
25. Cretney E, Kallies A, Nutt SL. Differentiation and function of Foxp3(+) effector regulatory T cells. Trends Immunol. 2013; 34(2):74-80.
26. Kitagawa Y, Sakaguchi S. Molecular control of regulatory T cell development and function. Curr Opin Immunol. 2017; 49:64-70.
27. Levine AG, Arvey A, Jin W, Rudensky AY. Continuous requirement for the TCR in regulatory T cell function. Nat Immunol. 2014; 15(11):1070-1078.
28. Spence A, Klementowicz JE, Bluestone JA, Tang Q. Targeting Treg signaling for the treatment of autoimmune diseases. Curr Opin Immunol. 2015; 37:11-20.
29. Biton J, Khaleghparast Athari S, Thiolat A, et al. In Vivo Expansion of Activated Foxp3+ Regulatory T Cells and Establishment of a Type 2 Immune Response upon IL-33 Treatment Protect against Experimental Arthritis. J Immunol Baltim Md 1950. 2016; 197(5):1708-1719.
30. Salomon BL, Leclerc M, Tosello J, Ronin E, Piaggio E, Cohen 438 JL. Tumor Necrosis Factor α and Regulatory T Cells in Oncoimmunology. Front Immunol. 2018; 9:444.
31. Koh AY, Priebe GP, Ray C, Van Rooijen N, Pier GB. Inescapable need for neutrophils as mediators of cellular innate immunity to acute Pseudomonas aeruginosa pneumonia. Infect Immun. 2009; 77(12):5300-5310.
32. Raschi E, Mazzarella A, Antonazzo IC, et al. Toxicities with Immune Checkpoint Inhibitors: Emerging Priorities From Disproportionality Analysis of the FDA Adverse Event Reporting System. Target Oncol. 2019; 14(2):205-221.

## Claims

1. A tumor necrosis factor receptor 2 (TNFR2) antagonist and/or composition enriched in lymphocytes and depleted of T regulatory (Treg) cells, for use in the prevention or treatment of an acute post-stress immunodepression.

2. A tumor necrosis factor receptor 2 (TNFR2) antagonist and/or composition enriched in lymphocytes and depleted of T regulatory (Treg) cells for use according to claim 1, wherein the acute post-stress immunodepression is sepsis.

3. The TNFR2 antagonist for use according to claim 1 or 2, wherein said antagonist is selected from the group consisting of a blocking anti-TNFR2 antibody or an antigen-binding fragment thereof having an antagonistic effect on TNFR2 upon binding.

4. The antigen-binding fragment for use according to claim 3, wherein said fragment is a Fab, Fab', F(ab)'2, scFv, (scFv')2, diabody, triabody, VH or VL domain.

5. The composition enriched in lymphocytes and depleted of Treg cells for use according to claim 1 or 2, wherein said composition is obtained by contacting *in vitro* a population of human cells comprising said lymphocytes from a human blood or bone marrow sample from a patient having sepsis with a TNFR2 antagonist that suppresses proliferation of Treg cells, thereby producing said composition enriched in lymphocytes and depleted of Treg cells.

6. The composition enriched in lymphocytes and depleted of Treg cells for use according to claim 5, wherein said composition is depleted of TNFR2pos Treg cells.
